(19) **Europäisches Patentamt / European Patent Office / Office européen des brevets**

(11) **EP 2 728 247 B1**

(12) **EUROPEAN PATENT SPECIFICATION**

(45) Date of publication and mention
of the grant of the patent:
**06.11.2019 Bulletin 2019/45**

(51) Int Cl.:
*F21V 8/00* (2006.01)   *A61B 5/00* (2006.01)
*G01N 21/17* (2006.01)

(21) Application number: **13180187.0**

(22) Date of filing: **13.08.2013**

(54) **OBJECT INFORMATION ACQUIRING APPARATUS**

Vorrichtung zur Erfassung von Objektinformationen

APPAREIL D'ACQUISITION D'INFORMATIONS SUR UN OBJET

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.09.2012 JP 2012194164**

(43) Date of publication of application:
**07.05.2014 Bulletin 2014/19**

(73) Proprietor: **Canon Kabushiki Kaisha
Tokyo 146-8501 (JP)**

(72) Inventors:
• **Yamamoto, Hiroshi**
**Tokyo, Tokyo 146-8501 (JP)**
• **Furukawa, Yukio**
**Tokyo, Tokyo 146-8501 (JP)**

(74) Representative: **TBK
Bavariaring 4-6
80336 München (DE)**

(56) References cited:
**WO-A1-2011/012274    WO-A1-2011/135820
JP-A- 2002 214 707**

EP 2 728 247 B1

**Description**

BACKGROUND OF THE INVENTION

Field of the Invention

The present invention relates to an object information acquiring apparatus.

Description of the Related Art

One method of obtaining an optical characteristic value in a living organism is photoacoustic tomography (PAT) which utilizes a property of ultrasonic waves of being less scattered than light in a living organism (Non Patent Literature 1: M. Xu, L. Wang "Photoacoustic imaging in biomedicine", Review of scientific instruments, 77, 041101(2006)). When pulsed light generated by a light source is irradiated on a living organism, the light propagates in the living organism while being diffused. When an absorber included in the living organism absorbs propagated light, an acoustic wave such as an ultrasonic wave is generated due to a photoacoustic effect. By receiving the ultrasonic wave with a probe and analyzing the received signal, an optical characteristic value distribution and, more particularly, a light absorption density distribution in the living organism can be obtained.

According to M. Xu, L. Wang "Photoacoustic imaging in biomedicine, Review of scientific instruments, 77, 041101(2006), sound pressure P of an ultrasonic wave obtained from an absorber in a living organism by light absorption according to PAT can be expressed by Equation (1) below.

$$P = \Gamma \cdot \mu_a \cdot \phi \ \ldots \ (1)$$

In Equation (1), $\Gamma$ denotes the Gruneisen coefficient which is an elasticity characteristic value obtained by dividing a product of a coefficient of volumetric expansion $\beta$ and the square of the speed of sound c by specific heat $C_p$. $\mu_a$ denotes an absorption coefficient of the absorber and $\phi$ denotes a light flux that is absorbed by the absorber.

As is apparent from Equation (1), sound pressure of an ultrasonic wave according to PAT is proportional to an amount of light that reaches the object. Therefore, in order to obtain a strong signal, the amount of the light that is irradiated on the object must be increased.

On the other hand, maximum permissible exposure (MPE) as a maximum value of irradiation density that is applied to a living organism is specified as a safety-related standard regarding lasers (JISC 6802). Uniform illumination is required in order to increase the amount of light irradiated on a living organism while taking MPE into consideration.

In addition, with PAT, in order to conduct a measurement over a wide range of a living organism, the living organism is desirably scanned by an illuminating unit and a receiver. In the case of a large light source such as a solid-state laser, it is difficult to perform a scan using the light source itself. Therefore, preferably, light emitted from the light source is transmitted by an optical fiber and a scan is performed using an outputting unit of the optical fiber. When the amount of light is large and light cannot be transmitted by a single optical fiber, a bundle fiber that is a bundle of optical fibers is preferably used.

Since a property of a bundle fiber is that light spreads after being outputted, a peripheral part has a smaller amount of light than a central part. While uniform beams can conceivably be produced by using a lens or the like between the bundle fiber and the living organism, this disadvantageously increases apparatus size. In particular, with PAT which uses a hand-held illuminating unit, an operating unit is desirably constituted by a minimum number of parts in order to reduce weight.

In the field of image display apparatuses, an example is disclosed in which an outputting unit of a bundle fiber branches into a plurality of equal sub-bundles (Patent Literature 1: Japanese Patent Application Laid-open No. 2002-214707). Accordingly, light emitted from a light source is transmitted by an optical fiber whose outputting unit branches into a plurality of sub-bundles, and by arranging the sub-bundles at positions corresponding to respective display elements and illuminating the display elements, a variation in amounts of light among the display elements is reduced and a high-quality image is obtained.

Patent Literature 1: Japanese Patent Application Laid-open No. 2002-214707

Non Patent Literature 1: M. Xu, L. Wang "Photoacoustic imaging in biomedicine", Review of scientific instruments, 77, 041101(2006)

**Prior art which is related to this field of technology can be found e.g. in document** JP 2002214707 A **disclosing an image display device, in document** WO 2011/012274 A1 **disclosing an imaging device and method for optoacoustic imaging of small animals, and in document** WO 2011/135820 A1 **disclosing a photoacoustic**

**measuring apparatus.**

SUMMARY OF THE INVENTION

The method disclosed in Japanese Patent Application Laid-open No. 2002-214707 has a problem in that when the outputting unit of the fiber and an illuminated region are close to each other, a dark portion that is not illuminated is created between regions illuminated by the sub-bundles and uniform illumination cannot be achieved. In addition, when the outputting unit of the fiber and an illuminated region are far from each other, there is a problem that overlapping of light is stronger in a central part than in a peripheral part of an illuminated region and uniform illumination cannot be achieved.

The present invention has been made in consideration of these problems. This present invention is developed to improve uniformity of light irradiated from a bundle fiber on an object.

The present invention in its first aspect provides an object information acquiring apparatus as specified in claims 1 to 4.

The present invention in its second aspect provides an object information acquiring apparatus as specified in claims 5 to 6.

The present invention in its third aspect provides an object information acquiring apparatus as specified in claim 7.

According to the present invention, the uniformity of light irradiated from a bundle fiber onto an object can be improved.

Further features of the present invention will become apparent from the following description of exemplary embodiments with reference to the attached drawings.

BRIEF DESCRIPTION OF THE DRAWINGS

FIG. 1 is a diagram showing a typical embodiment of the present invention;
FIGS. 2A to 2E are diagrams showing light outputted from a sub-bundle;
FIGS. 3A to 3E are diagrams showing an irradiation density distribution according to a first example;
FIGS. 4A to 4D are diagrams illustrating a conventional method;
FIGS. 5A to 5E are diagrams illustrating a second example;
FIGS. 6A to 6D are diagrams illustrating a third example;
FIGS. 7A to 7D are diagrams illustrating a fourth example;
FIGS. 8A to 8D are diagrams showing an irradiation density distribution according to a conventional method;
FIG. 9 is a diagram illustrating a fifth example; and
FIGS. 10A and 10B are diagrams illustrating a sixth example.

DESCRIPTION OF THE EMBODIMENTS

Hereinafter, a preferred embodiment of the present invention will be described with reference to the drawings. However, dimensions, materials, shapes, relative arrangements, and the like of components described below are to be modified as appropriate depending on configurations and various conditions of apparatuses to which the invention is applied, and are not intended to limit the scope of the invention to the following description.

The present invention can be favorably applied to an illuminating apparatus which irradiates light using a bundle fiber. In particular, by applying the present invention to an object information acquiring apparatus including such an illuminating apparatus, the uniformity of light irradiated on an object can be improved. Such object information acquiring apparatuses include apparatuses which irradiate light (electromagnetic waves) on an object and receive acoustic waves generated inside the object, and which acquire object information in the form of image data.

Object information that is acquired by an apparatus utilizing a photoacoustic effect represents a generation source distribution of acoustic waves generated by light irradiation, an initial sound pressure distribution inside an object, or a light energy absorption density distribution, an absorption coefficient distribution, or a concentration distribution of a tissue-forming substance that is derived from the initial sound pressure distribution. Examples of a concentration distribution of a substance include an oxygen saturation distribution and an oxygenated/reduced hemoglobin concentration distribution.

Acoustic waves as described in the present invention are typically ultrasonic waves and include elastic waves that are referred to as sound waves, ultrasonic waves, or acoustic waves. An acoustic wave generated by a photoacoustic effect is referred to as a photoacoustic wave or a light ultrasonic wave. An acoustic detector (for example, a probe) receives acoustic waves generated or reflected inside an object.

Hereinafter, an object information acquiring apparatus according to an embodiment of the present invention and, in particular, a configuration of an illuminating apparatus that is a feature of the present invention will be described.

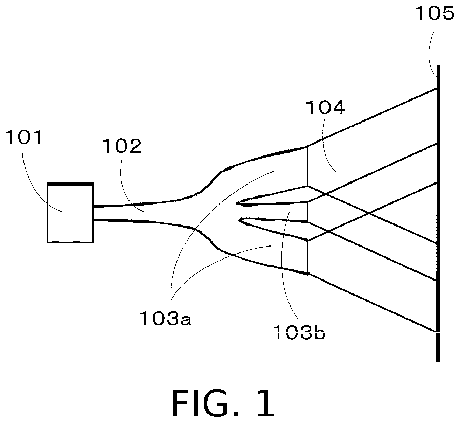

FIG. 1 is a diagram showing an overview configuration of an illuminating apparatus according to a typical embodiment of the present invention. The illuminating apparatus comprises a light source 101, a bundle fiber 102, and sub-bundles 103. The sub-bundles 103 include a sub-bundle 103a that is made up of a large number of fibers and a sub-bundle 103b that is made up of a small number of fibers. Outgoing light 104 is irradiated from the sub-bundle on a light-irradiated surface 105. In addition, the object information acquiring apparatus comprises various components other than the illuminating apparatus. Hereinafter, the respective components will be described.

(Light source)

When the object is a living organism, the light source irradiates light with a wavelength that is absorbed by a specific component among components that constitute the living organism. The light source may be integrally provided with the living organism image acquiring apparatus according to the present embodiment, or the light source may be separated and provided as a separate body. A pulse width is preferably around 10 to 50 nanoseconds in order to generate photoacoustic waves in an efficient manner.

While a laser capable of producing a large output is favorably used as the light source, a light-emitting diode, a flash lamp, or the like can be used instead of a laser. Various lasers can be used including a solid-state laser, a gas laser, a dye laser, and a semiconductor laser. Timing, waveform, intensity, and the like of irradiation are controlled by a light source controller.

Moreover, the light source controller may be integrated with the light source. The wavelength of the light source used in the present invention is desirably a wavelength that allows light to propagate to the inside of an object. Specifically, when the object is a living organism, the wavelength is equal to or more than 500 nm and equal to or less than 1200 nm.

(Bundle fiber)

The bundle fiber is constructed by bundling together a plurality of optical fibers. An optical fiber has a core made of silica glass or the like. Specifically, for example, the core has a diameter of 190 $\mu$m. Light from the light source enters from an incidence section of the bundle fiber and is transmitted by the respective fibers.

An output side of the bundle fiber branches into a plurality of sub-bundles. Each sub-bundle is provided with an outputting edge for outputting the transmitted light. The outputting edge of each sub-bundle is fixed so that end faces of the outputting edges are aligned in an outputting unit. A plane of the outputting unit on which sub-bundle end faces are aligned form an outputting plane. Light outputted from the plurality of sub-bundle end faces on the outputting plane overlap each other to illuminate an object. A sub-bundle in a central part corresponds to the first sub-bundle and a sub-bundle in a peripheral part corresponds to the second sub-bundle.

When the same sub-bundle is aligned at equal intervals, since overlapping of light in the peripheral part is weaker than overlapping of light in the central part, uniformity is low. In consideration thereof, in order to increase the amount of light in the peripheral part to improve uniformity, the bundle fiber according to the present invention is configured such that the density of an optical fiber in the peripheral part (the sub-bundle 103a) is greater than the density of an optical fiber in the central part (the sub-bundle 103b) in the outputting unit. However, if the outputting edge of a sub-bundle and the light-irradiating plane are too close to each other, light does not overlap and the effect of the present invention cannot be produced.

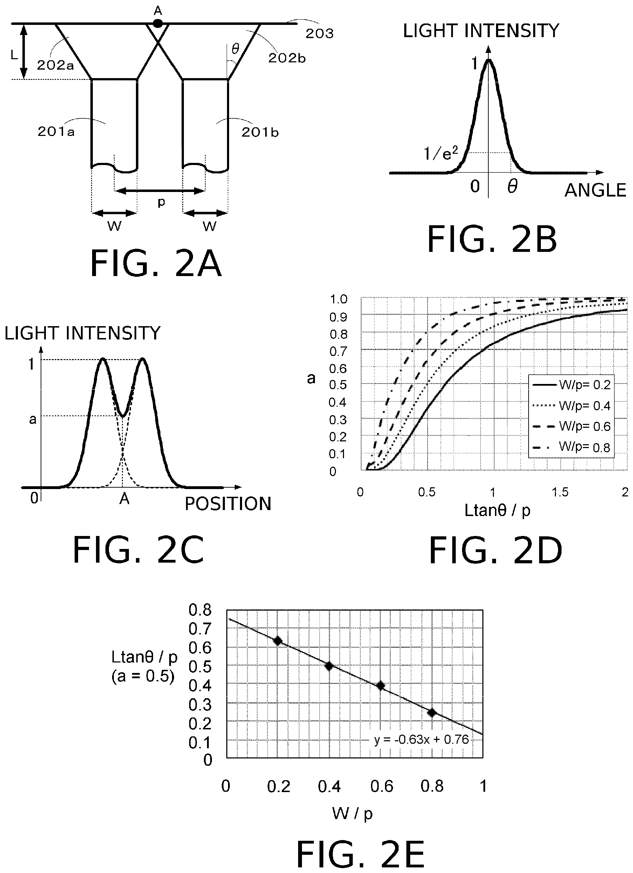

In consideration thereof, a range in which the effect of the present invention can be produced will be described with reference to FIG. 2. FIG. 2A shows how light 202a and light 202b outputted from two sub-bundles 201a and 201b overlap each other on a light-irradiated surface 203. A point A represents a point on the light-irradiated surface 203 which is straight in front between the sub-bundles 201a and 201b. In this case, L denotes a distance from the outputting end face of a sub-bundle to the irradiated surface, p denotes a minimum pitch between two adjacent sub-bundles, and W denotes a width of a sub-bundle.

FIG. 2B shows a distribution of light outputted from a single fiber as a contrast between angle and intensity (an angular distribution of light intensity). In this case, a spread angle of light outputted from a fiber is defined as an angle $\theta$ over which intensity drops from a maximum intensity to $1/e^2$. A degree of overlapping of light outputted from the sub-bundles 201a and 201b is expressed as a ratio a between maximum light intensity and light intensity at the point A on the irradiated surface. FIG. 2C is a diagram showing overlapping of light outputted from the two sub-bundles. FIG. 2D is a diagram showing a relationship between a and $L \cdot \tan\theta/p$ when W/p is varied from 0.2 to 0.8.

In addition, FIG. 2E is a diagram showing a relationship between $L \cdot \tan\theta/p$ and W/p at a = 0.5. From FIG.

2E, the relationship between $L \cdot \tan\theta / p$ and W/p can be almost linearly approximated, and when a range where the present invention becomes significant is assumed to be a ≥ 0.5, then the relationship can be expressed as Equation (2) below.

$$L \cdot \tan\theta \geq 0.76p - 0.63W \quad \ldots \quad (2)$$

In consideration thereof, the following examples have been configured so as to satisfy this condition.

(Object information acquiring apparatus)

When a living organism is the object, the object information acquiring apparatus according to the present embodiment is a living organism image acquiring apparatus which calculates information from the living organism as image data. However, the object information acquiring apparatus may be used on an object other than a living organism. As basic hardware components, the object information acquiring apparatus comprises a light source, a bundle fiber, a probe which receives acoustic waves, and a processor which performs image reconstruction.

Pulsed light emitted from the light source is transmitted by the bundle fiber and irradiated on a living organism. When a part of the energy of light propagated inside the living organism is absorbed by a light absorber (which eventually becomes a sound source) such as blood, an acoustic wave is generated by a thermal expansion of the light absorber. The acoustic wave is received by the probe and becomes an electric signal which is then transmitted to the processor. Based on the electric signal, the processor generates optical characteristic value distribution information from the living organism (image reconstruction). The optical characteristic value distribution information is not limited to a particular format. A format of the optical characteristic value distribution information can be arbitrarily determined based on a measurement objective, an apparatus configuration, and the like including two-dimensional and three-dimensional formats.

(Probe)

The probe receives an acoustic wave generated on a surface of the living organism or inside the living organism due to the pulsed light. Therefore, the probe is capable of converting the acoustic wave into an electric signal (received signal) that is an analog signal. Any kind of probe may be used including a probe using a piezoelectric phenomenon, a probe using resonance of light, and a probe using a variation in capacitance as long as the probe is capable of receiving acoustic wave signals. Favorably, the probe according to the present embodiment typically has a plurality of receiving elements arranged one-dimensionally or two-dimensionally. Using such multidimensionally-arranged elements enables acoustic waves to be simultaneously received at a plurality of locations to reduce measurement time. When there is only one receiving element, a scan may be performed using the probe to receive acoustic waves at a plurality of positions.

The object information acquiring apparatus desirably comprises a converter which converts an electric signal obtained by the probe from an analog signal to a digital signal and a circuit which amplifies the electric signal. When a plurality of received signals is obtained from the probe, desirably, a plurality of signals is simultaneously processed. Accordingly, the time required to form an image can be reduced. The converted signal is stored in a memory.

(Processor)

The processor uses the signal stored in the memory to form data related to optical characteristic value distribution information such as an initial sound pressure distribution of acoustic waves. For example, time-domain back projection can be used to form the optical characteristic value distribution. For example, an information processing device or a circuit which is operated by a program can be used as the processor.

<First example>

In the present example, an example where the density of fibers contained in a sub-bundle in a central part is smaller than the density of fibers contained in a sub-bundle in a peripheral part will be described.

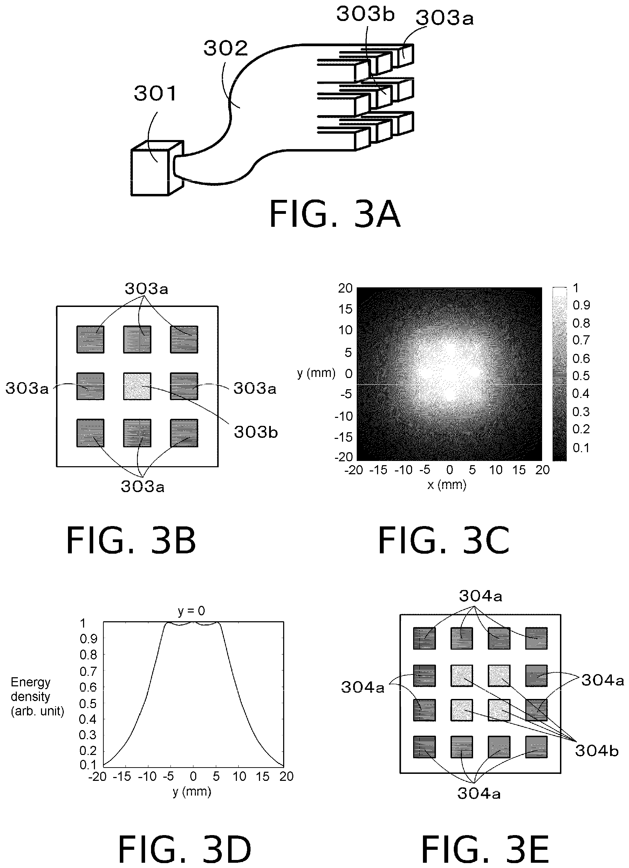

FIG. 3A is an overall view of an illuminating apparatus comprising a light source and a bundle fiber according to the present example. FIG. 3B is a diagram showing a shape of a fiber outputting unit according to the present example. The illuminating apparatus transmits light from a light source 301 that is a solid-state laser with an emission wavelength of 800 nm using a bundle fiber 302. A titanium-sapphire laser was used as the light source 301.

A diameter of a core of a fiber strand is 190 μm. In the incidence section, fiber strands are approximately hexagonal close-packed. The outputting unit of the bundle fiber 302 branches into nine 1 mm [sq] sub-bundles 303. The nine sub-bundles are arranged in a 3 × 3 array at an equal pitch p of 6 mm between two adjacent sub-bundles.

A feature of the present example is that the numbers of optical fibers contained in the respective sub-bundles differ between the central part and the peripheral part. Specifically, if the number of fibers contained per unit area of the sub-bundle 303a in the peripheral part is 1, then the number of fibers contained per unit area of the sub-bundle 303b in the central part is 0.3. Light outputted from each fiber can be approximated to a Gaussian distribution. A measurement of a spread angle $\theta$ of light from a single optical fiber in the present example resulted in $\tan\theta = 0.1$. In this case, an angle over which intensity drops from a maximum intensity to $1/e^2$ is defined as $\theta$.

FIG. 3C shows an irradiation density distribution at the light-irradiated surface when a distance L from a central sub-bundle to the light-irradiated surface is 100 mm. In addition, FIG. 3D shows an irradiation density distribution of a cross section taken by cutting a center of the irradiation density distribution shown in FIG. 3C in a lateral direction.

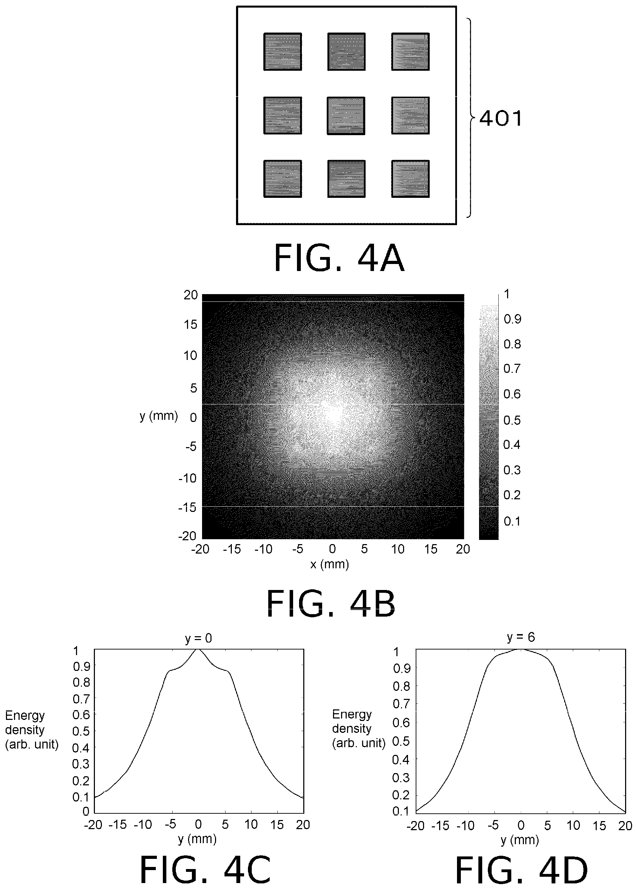

On the other hand, an example of a conventional illuminating apparatus as a target for comparison is shown in FIG. 4. FIG. 4A shows a case where the numbers of optical fibers per unit area contained in nine sub-bundles 401 are all equal. In addition, FIG. 4B shows an irradiation density distribution at the light-irradiated surface in the case of FIG. 4A. Furthermore, FIG. 4C shows an irradiation density distribution of a cross section taken by cutting a center of FIG. 4B in a lateral direction.

A comparison of FIG. 3D showing a cross section according to the present example with FIG. 4C showing a conventional cross section reveals that uniformity of light is improved and a more uniform illumination distribution can be obtained by the present example. This is because the numbers of fibers have been varied depending on positions of the respective sub-bundles. In other words, arranging light from the peripheral part with high irradiation density to be diagonally incident even to the irradiated surface which opposes the central part can compensate for the low irradiation density in the central part and, as a whole, an approximately uniform light irradiation can be realized.

While an example where square sub-bundles are arranged at equal intervals in a 3 × 3 array has been given in the description above, the present invention is not limited thereto. As an example, a 4 × 4 arrangement of square sub-bundles is shown in FIG. 3E. The number of optical fibers contained in the four sub-bundles 304b in the central part is smaller than the number of optical fibers contained in the 12 sub-bundles 304a on the outer side (peripheral part). Even in this case, diagonally-incident light from the peripheral part compensates for the low irradiation density in the central part and uniformity of light on the irradiated surface is improved.

In addition to sub-bundle arrangements with the same number of vertical and horizontal sub-bundles as shown in FIG. 3, the present invention can also be applied to other sub-bundle arrangements such as 4 × 5 and 5 × 7. Even in such cases, by setting the density of optical fibers near the center of the outputting unit lower than the density of core optical fibers in the peripheral part of the outputting unit, light can be uniformly irradiated. In addition, while the shapes of the fibers are all the same, core areas per unit area may be varied by using optical fibers with different core diameters between sub-bundles in the central part and sub-bundles in the peripheral part . Furthermore, even when the sub-bundles are circularly arranged, the present invention can be applied by relatively suppressing the amount of light in the central part in assumption of the central part and the peripheral part.

<Second example>

In the present example, an example will be described which produces a more uniform illumination distribution by varying the density of optical fibers contained in sub-bundles depending on location even in the peripheral part. Specifically, when the sub-bundle array is square, the density of optical fibers contained in a sub-bundle corresponding to a side of the square is set lower than the density of optical fibers contained in a sub-bundle at a corner (vertex) of the square.

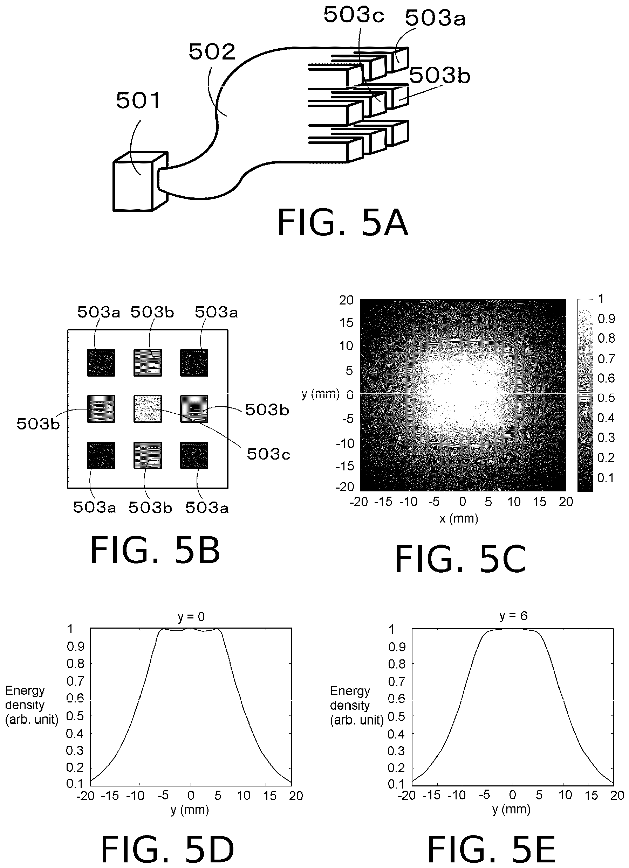

FIG. 5A is an overall view of an illuminating apparatus comprising a light source and a bundle fiber according to the present example. FIG. 5B is a diagram showing a shape of a fiber outputting unit according to the present example. The illuminating apparatus comprises a light source 501 and a bundle fiber 502.

The outputting unit of the bundle fiber 502 branches into nine 1 mm [sq] sub-bundles in a similar manner to the first example. The nine sub-bundles are arranged in a 3 × 3 array at an equal pitch p of 6 mm between two adjacent sub-bundles.

A feature of the present example is that the numbers of optical fibers contained in the respective sub-bundles vary depending on positions of the sub-bundles. In other words, if the number of optical fibers per unit area of a sub-bundle 503a in the peripheral part and at a vertex of a square is five, then the numbers of optical fibers per unit area of a sub-bundle 503b on a side portion of the peripheral part and a central part 503c are, respectively, three and one. Light outputted from each optical fiber can be approximated to a Gaussian distribution. In the present example, a spread angle $\theta$ of light from a single optical fiber was $\tan\theta = 0.1$. In this case, an angle over which intensity drops from a maximum intensity to $1/e^2$ is defined as $\theta$.

FIG. 5C shows an irradiation density distribution at the light-irradiated surface when a distance L from a central sub-bundle to the light-irradiated surface is 100 mm. In addition, FIGS. 5D and 5E respectively show an irradiation density distribution of a cross section taken by cutting a center of the irradiation density distribution shown in FIG. 5C in a lateral direction and an irradiation density distribution of a cross section taken by cutting a point that is separated upward by one pitch (6 mm) from the center of the irradiation density distribution shown in FIG. 5C in a lateral direction.

As a target for comparison, FIG. 4D shows an irradiation density distribution of a cross section taken by cutting a point that is separated upward by one pitch (6 mm) from the center of the irradiation density distribution shown in FIG. 4B in a lateral direction. When comparing FIG. 5D with FIG. 4C, irradiation density distributions of cross sections at the center of the irradiation density distributions are similar. Furthermore, when comparing FIG. 5E with FIG. 4D, the irradiation density distribution of a cross section at a point that is separated upward by one pitch (6 mm) from the center of the irradiation density distribution has improved uniformity in the present example. This is due to an improvement in the uniformity of the amount of light on the irradiated surface by relatively lowering irradiation density at side portions which receive diagonally-incident light from both sides among the peripheral part and raising irradiation density at vertices which only receive diagonally-incident light from a side portion on one side among the peripheral part.

From the above, by varying the density of optical fibers contained in sub-bundles depending on locations of the sub-bundles even in the peripheral part as in the present example, a more uniform illumination distribution can be obtained. Moreover, while an example where an array of square sub-bundles is arranged in a square has been described in the present example, this example is not restrictive. In addition, the sub-bundles may be arranged in a hexagonal close-packed structure.

Examples of non-square arrangements of sub-bundles include polygonal sub-bundle arrangements such as a triangle or a hexagon. In such cases, the numbers of optical fibers per unit area of sub-bundles in a central part, a side part, and a vertex part of the polygon need only satisfy (central part) < (side part) < (vertex part) .

<Third example>

In the present example, an example will be described in which the numbers of optical fibers per unit area of respective sub-bundles are the same and a uniform illumination distribution is obtained by varying areas of the respective sub-bundles according to position.

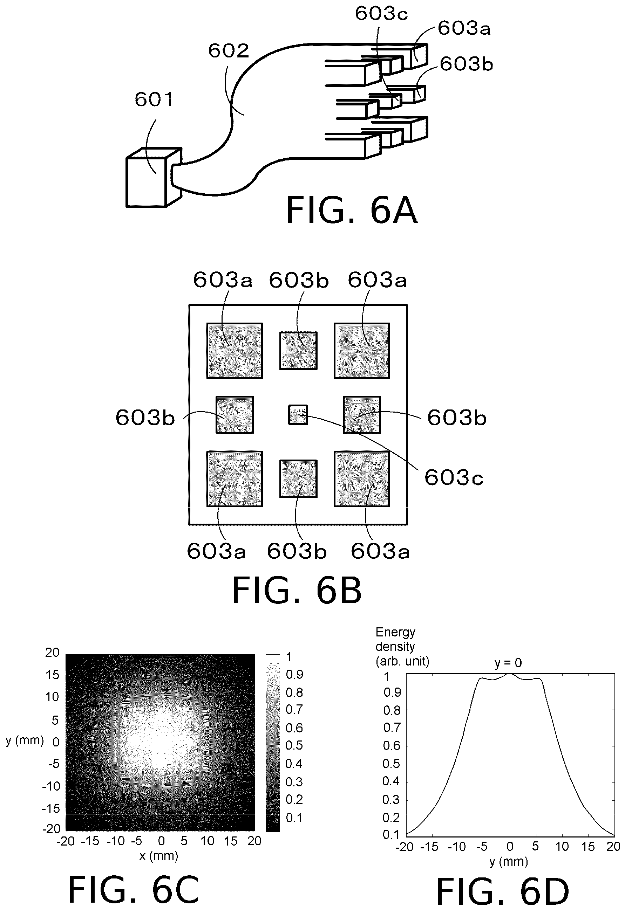

FIG. 6A is an overall view of an illuminating apparatus comprising a light source and a bundle fiber according to the present example. FIG. 6B is a diagram showing a shape of a fiber outputting unit according to the present example. The illuminating apparatus comprises a light source 601 and a bundle fiber 602.

The outputting unit of the bundle fiber 602 branches into nine sub-bundles. While the number of optical fibers per unit area of the respective sub-bundles is the same, sizes of the sub-bundles vary depending on an arrangement of the sub-bundles and the closer to center, the smaller the sub-bundle. A size of a sub-bundle 603a near a vertex in the peripheral part is 1.2 mm [sq], a size of a sub-bundle 603b of a side part in the peripheral part is 1 mm [sq], and a size of a sub-bundle 603c in the central part is 0.7 mm [sq].

FIG. 6C shows an irradiation density distribution at the light-irradiated surface when a distance from a central sub-bundle to the light-irradiated surface is 100 mm. In addition, FIG. 6D shows an irradiation density distribution of a cross section taken by cutting a center of the irradiation density distribution shown in FIG. 6C in a lateral direction. As is apparent from a comparison of FIGS. 6D and 4C, even if the number of optical fibers per unit area in the respective sub-bundles is set the same, a more uniform illumination distribution can be obtained by adopting an arrangement in which sizes of the sub-bundles are varied.

The illuminating apparatus according to the present example is advantageous in that the illuminating apparatus is easier to manufacture than a system where the number of optical fibers per unit area is varied as in the first and second examples.

<Fourth example>

Examples where an irradiation density or an area of an outputting edge varies among sub-bundles have been described for the first to third examples. In the present example, an example will be described in which sub-bundles with the same irradiation density or area are arranged so that density in a central part is lower than density in a peripheral part.

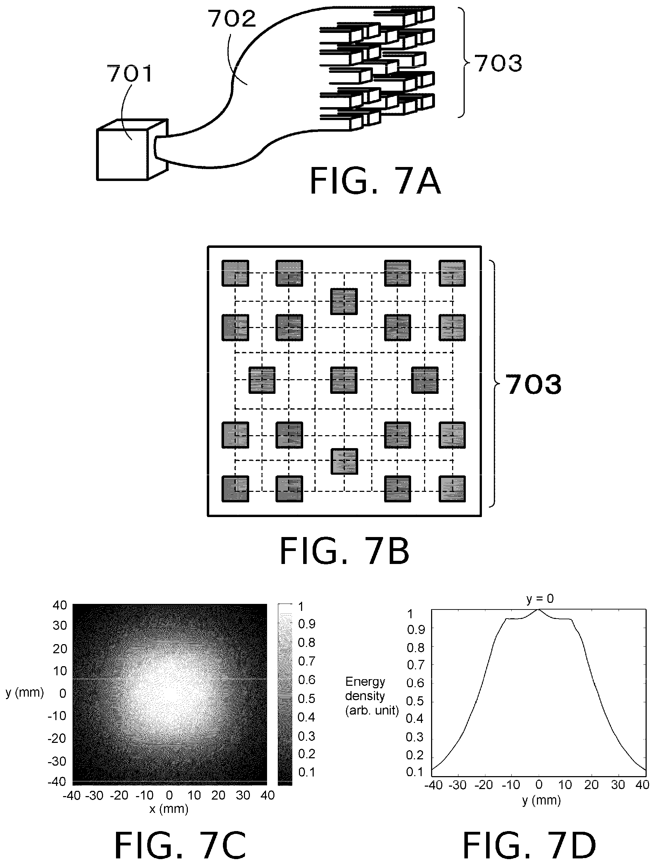

FIG. 7A is an overall view of an illuminating apparatus comprising a light source and a bundle fiber according to the present example. FIG. 7B is a diagram showing a shape of a fiber outputting unit according to the present example. The illuminating apparatus comprises a light source 701 and a bundle fiber 702.

The outputting unit of the bundle fiber 702 branches into 21 sub-bundles 703 (1 mm [sq]). The 21 sub-bundles 703 are arranged as shown in FIG. 7B. A pitch of the dotted-line mesh is set to 4 mm. The number of optical fibers contained in the respective sub-bundles is the same. As shown in the diagram, outputting edges are sparse in the central part and dense in the peripheral part. Furthermore, among the peripheral part, vertex portions are denser than side portions.

FIG. 7C shows an irradiation density distribution at the light-irradiated surface when a distance from a central sub-bundle to the light-irradiated surface is 100 mm. In addition, FIG. 7D shows an irradiation density distribution of a cross section taken by cutting a center of the irradiation density distribution shown in FIG. 7C in a lateral direction.

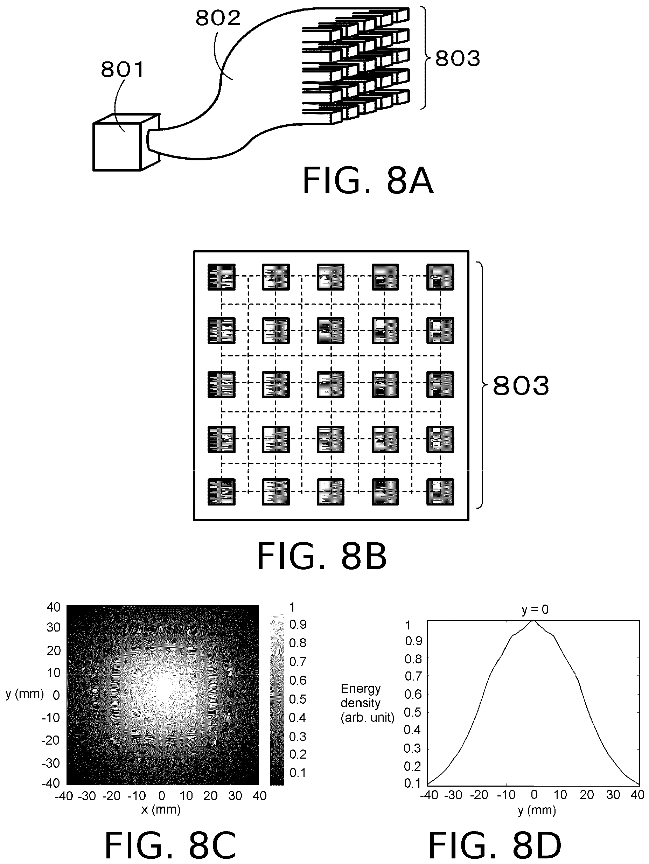

As a target for comparison, FIG. 8 shows an example where respective sub-bundles are arranged at equal intervals. The outputting edges in the illuminating apparatus shown in FIG. 8A are arranged such that the density of sub-bundles is the same at center and in the peripheral part as shown in FIG. 8B. In addition, FIG. 8C shows an irradiation density distribution on the light-irradiated surface, and FIG. 8D shows an irradiation density distribution of a cross section taken by cutting a center of FIG. 8C in a lateral direction. A pitch of the dotted-line mesh shown in FIG. 8B is set to 4 mm.

As is apparent from a comparison of FIGS. 7D and 8D, by arranging the sub-bundles so that the densities of the sub-bundles differ between the center and the peripheral part of the outputting unit, a more uniform illumination distribution can be obtained. This is because the amount of diagonally-incident light from bundle fibers corresponding to adjacent regions is greater in the central part, and even if outputting edges are sparsely arranged in the central part, a total amount of light becomes equal to that of the peripheral part. Conversely, in the peripheral part, even if the outputting edges are densely arranged, a total amount of light becomes more or less equal to that of the central part because there is a smaller amount of incident light.

Although an example where sub-bundles are arranged in a square shape has also been described in the present example, this arrangement is not restrictive. Specifically, sub-bundles may be arranged in a polygonal shape such as a triangle or a hexagon. In such cases, the numbers of arranged outputting edges of sub-bundles in a central part, a side part, and a vertex part of the polygon need only satisfy (central part) < (side part) < (vertex part).

<Fifth example>

While an illuminating apparatus has been described in the first to fourth examples, in the present example, an example will be described where a uniform illumination distribution is realized without inserting a lens system between a bundle fiber and a living organism in an object information acquiring apparatus.

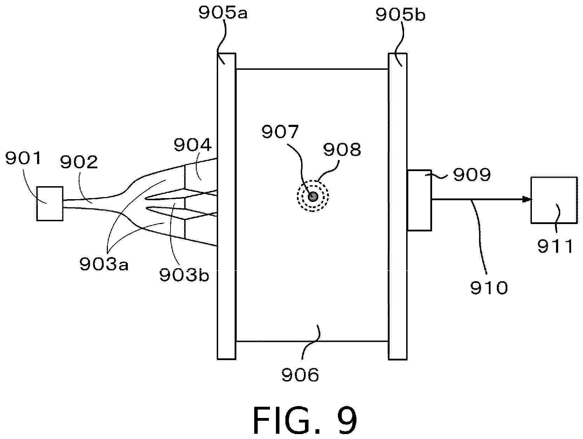

FIG. 9 shows an object information acquiring apparatus according to the present example. Light from a light source 901 is transmitted by a bundle fiber 902 and outputted from sub-bundles 903a and 903b. In this case, the sub-bundles were arranged in a similar manner to the first example. Among the sub-bundles, the sub-bundle 903a has a smaller number of optical fibers per unit area, and the sub-bundle 903b has a larger number of optical fibers per unit area.

Light 904 outputted from a sub-bundle illuminates a living organism 906 that is held by a holding plate 905a on the side of the bundle fiber 902 and a holding plate 905b on the opposite side of the bundle fiber 902. Desirably, the holding plate 905a readily transmits light while the holding plate 905b readily transmits acoustic waves and has an acoustic impedance that is close to the acoustic impedance of a living organism. For example, the holding plate 905a may be made of acryl and the holding plate 905b may be made of polymethylpentene. In the present example, acryl and polymethylpentene both have a thickness of 10 mm. Acryl has a refractive index of 1.49. In order to secure an optical path length of 100 mm from the fiber outputting edge to the living organism in a similar manner to the first example, a distance from the fiber outputting edge to the acryl plate was set to 85.1 mm.

The illuminated light is diffused in the living organism 906, and an acoustic wave 908 is generated when the diffused light is absorbed by an absorber 907. The acoustic wave 908 is propagated in the living organism 906 that is an object, and a part of the acoustic wave 908 is received by a probe 909. A received signal 910 is sent to a processor 911 and optical characteristic value distribution information in the living organism is formed. The sub-bundles 903a and 903b and the probe 909 are movable in a two-dimensional direction that is parallel to the holding plate 905.

By adopting the configuration described above, a variation in light intensity distribution among different light-irradiating positions can be reduced in a similar manner to the first example. As a result, a photoacoustic signal can be obtained more efficiently.

<Sixth example>

While a system in which a holding plate is scanned by a sub-bundle and a probe has been described in the fifth example, in the present example, a hand-held object information acquiring apparatus in which a sub-bundle and a probe are manually moved on a living organism will be described.

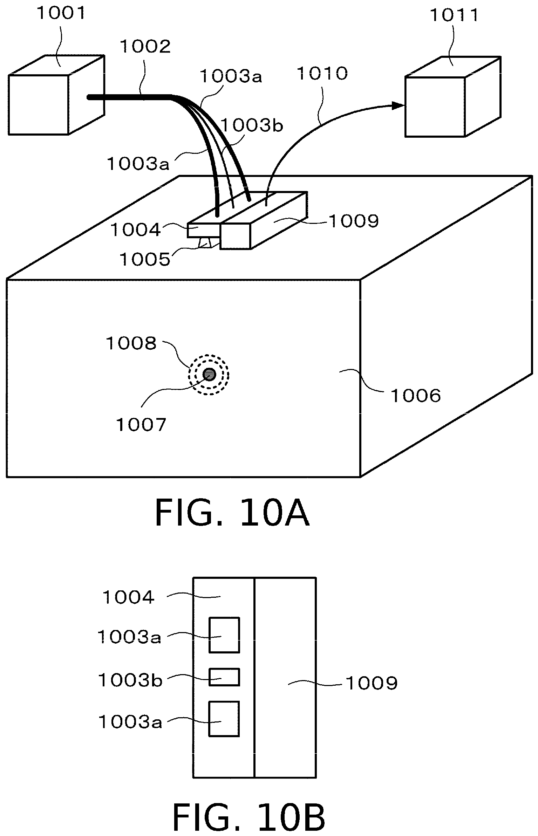

FIG. 10A shows an object information acquiring apparatus according to the present example. Light from a light source 1001 is transmitted by a bundle fiber 1002 and outputted from sub-bundles 1003a and 1003b. Among the sub-bundles, the sub-bundle 1003a has a smaller number of optical fibers per unit area, and the sub-bundle 1003b has a larger number of optical fibers per unit area. The sub-bundles 1003a and 1003b are integrated in an outputting unit 1004.

When performing a measurement, the outputting unit 1004 is brought into contact with a living organism 1006 that is an object and light 1005 illuminates the living organism 1006. The illuminated light is diffused in the living organism 1006, and an acoustic wave 1008 is generated when the diffused light is absorbed by an absorber 1007. The acoustic wave 1008 is propagated in the living organism 1006, and a part of the acoustic wave 1008 is received by a probe 1009. In this case, the probe 1009 is integrated with the outputting unit 1004 and can be used to manually scan the living organism 1006.

FIG. 10B is a diagram of the integrated outputting unit 1004 and probe 1009 as seen from the side of the living organism 1006. A received signal 1010 that is received by the probe 1009 is sent to a processor 1011 and an optical characteristic value distribution inside the living organism is formed.

While light irradiated on a living organism is desirably uniform as described earlier, in the case of a hand-held apparatus, the number of parts of an illuminating system is desirably minimized in order to reduce weight of an operating unit. Since the illuminating system described in the present example does not use an optical system for uniform illumination between the bundle fiber and the living organism, the illuminating system can be constructed using a minimum number of parts. Moreover, while a configuration which does not use a lens between an outputting unit and a living organism has been described in the present example, a more uniform optical system can be obtained by using an optical part such as a diffuser plate. In addition, a cover glass or the like can also be provided on an outputting edge of a bundle fiber.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

An object information acquiring apparatus is used that includes: a light source; a bundle fiber including a plurality of optical fibers; and a processor using an acoustic wave that is generated when light is irradiated on an irradiated surface of an object from an outputting unit of the bundle fiber, and acquiring information from the object, wherein outputting edges of a plurality of sub-bundles, each sub-bundle including a plurality of optical fibers, are arranged in the outputting unit of the bundle fiber, the plurality of sub-bundles include a first sub-bundle arranged in a central part of the outputting unit and a second sub-bundle arranged in a peripheral part of the outputting unit, and the number of optical fibers per unit area of the first sub-bundle is smaller than the number of optical fibers per unit area of the second sub-bundle.

## Claims

1. An object information acquiring apparatus comprising:

   a light source (101);
   a bundle fiber (102) including a plurality of optical fibers; and
   a processor using an acoustic wave that is generated when light from the light source is irradiated on an irradiated surface of an object from an outputting unit of the bundle fiber, and acquiring information from the object, wherein the plurality of optical fibers are arranged so that an amount of light per unit area on the irradiated surface when light is irradiated is approximately uniform, wherein
   outputting edges of a plurality of sub-bundles, each sub-bundle including a plurality of optical fibers, are arranged in the outputting unit of the bundle fiber,
   the plurality of sub-bundles include a first sub-bundle (103b) arranged in a central part of the outputting unit and a second sub-bundle (103a) arranged in a peripheral part of the outputting unit,
   **characterized in that**
   the number of optical fibers per unit area of the first sub-bundle is smaller than the number of optical fibers per unit area of the second sub-bundle.

2. The object information acquiring apparatus according to claim 1, wherein the outputting edges of the plurality of sub-bundles have a same size and are arranged at a same pitch in the outputting unit.

3. The object information acquiring apparatus according to claim 2, wherein the outputting edges of the plurality of sub-bundles are arranged in a polygonal shape, the second sub-bundle includes a side sub-bundle that is arranged on a side of the polygon and a vertex sub-bundle that is arranged on a vertex of the polygon, and the numbers of optical fibers per unit area of the plurality of sub-bundles are in an order expressed as:

   vertex sub-bundle > side sub-bundle > the first sub-bundle.

4. The object information acquiring apparatus according to claim 3, wherein

when L denotes a distance from the outputting edge of the sub-bundle to the irradiated surface, $\theta$ denotes an angle over which intensity of light irradiated from the optical fiber drops from a maximum intensity to $1/e^2$, p denotes a minimum pitch between two adjacent sub-bundles, and W denotes a width of a sub-bundle, then

$$L\cdot\tan\theta \geq 0.76p - 0.63W$$

is satisfied.

5. An object information acquiring apparatus comprising:

a light source (601);
a bundle fiber (602) including a plurality of optical fibers; and
a processor using an acoustic wave that is generated when light from the light source is irradiated on an irradiated surface of an object from an outputting unit of the bundle fiber, and acquiring information from the object, wherein the plurality of optical fibers are arranged so that an amount of light per unit area on the irradiated surface when light is irradiated is approximately uniform, wherein
outputting planes of a plurality of sub-bundles, each sub-bundle including a plurality of optical fibers, are arranged in the outputting unit of the bundle fiber,
the plurality of sub-bundles include a first sub-bundle (603c) arranged in a central part of the outputting unit and a second sub-bundle (603a, 603b) arranged in a peripheral part of the outputting unit,
**characterized in that**
an area of the outputting plane of the first sub-bundle is smaller than an area of the outputting plane of the second sub-bundle.

6. The object information acquiring apparatus according to claim 5, wherein
the number of optical fibers per unit area is the same among the plurality of sub-bundles.

7. An object information acquiring apparatus comprising:

a light source (701);
a bundle fiber (702) including a plurality of optical fibers; and
a processor using an acoustic wave that is generated when light from the light source is irradiated on an irradiated surface of an object from an outputting unit of the bundle fiber, and acquiring information from the object, wherein the plurality of optical fibers are arranged so that an amount of light per unit area on the irradiated surface when light is irradiated is approximately uniform, wherein
outputting edges of a plurality of sub-bundles (703), each sub-bundle including a plurality of optical fibers, are arranged in the outputting unit of the bundle fiber,
the plurality of sub-bundles include plural first sub-bundles arranged in a central part of the outputting unit and plural second sub-bundles arranged in a peripheral part of the outputting unit,
**characterized in that**
the outputting edges of the first sub-bundles are more sparsely arranged than the outputting edges of the second sub-bundles in the outputting unit.

**Patentansprüche**

1. Objektinformationserhaltevorrichtung mit:

einer Lichtquelle (101);
einem Faserbündel (102) mit einer Vielzahl von optischen Fasern; und
einem Prozessor, der eine akustische Welle, die erzeugt wird, wenn Licht von der Lichtquelle eine bestrahlte Oberfläche eines Objekts aus einer Ausgabeeinheit des Faserbündels bestrahlt, verwendet, und eine Information aus dem Objekt erhält, wobei
die Vielzahl der optischen Fasern so angebracht sind, dass eine Lichtmenge pro Einheitsfläche der bestrahlten Oberfläche, wenn sie mit Licht bestrahlt wird, ungefähr einheitlich ist, wobei
Ausgabekanten einer Vielzahl von Unterbündeln in der Ausgabeeinheit des Faserbündels angebracht sind, wobei jedes Unterbündel eine Vielzahl von optischen Fasern enthält,
die Vielzahl der Unterbündel ein erstes Unterbündel (103b), das in einem zentralen Teil der Ausgabeeinheit angebracht ist, und ein zweites Unterbündel (103a), das in einem peripheren Teil der Ausgabeeinheit angebracht ist, enthält,
**dadurch gekennzeichnet, dass**
die Anzahl der optischen Fasern pro Einheitsfläche des ersten Unterbündels kleiner als die Anzahl der optischen Fasern pro Einheitsfläche des zweiten Unterbündels ist.

2. Objektinformationserhaltevorrichtung nach Anspruch 1, wobei
die Ausgabekanten der Vielzahl der Unterbündel eine gleiche Größe haben und mit einem gleichen Abstand in der Ausgabeeinheit angebracht sind

3. Objektinformationserhaltevorrichtung nach Anspruch 2, wobei

die Ausgabekanten der Vielzahl der Unterbündel in einer polygonalen Form angebracht sind,
das zweite Unterbündel ein Seitenunterbündel, das auf einer Seite des Polygons angebracht ist, und ein Eckenunterbündel, das auf einer Ecke des Polygons angebracht ist, enthält, und
die Anzahl der optischen Fasern pro Einheitsfläche der Vielzahl der Unterbündel in einer Ordnung sind, die ausgedrückt wird durch:

$$\text{Eckenunterbündel} > \text{Seitenunterbündel} > \text{das erste Unterbündel.}$$

4. Objektinformationserhaltevorrichtung nach Anspruch 3, wobei,
wenn L einen Abstand von der Ausgabekante des Unterbündels zu der bestrahlten Fläche bezeichnet, $\theta$ einen Winkel, über den eine Intensität des Lichts, das aus der optischen Faser bestrahlt wird, von einer Maximalintensität zu $1/e^2$ abfällt, bezeichnet, p einen minimalen Abstand zwischen zwei benachbarten Unterbündeln bezeichnet, und W eine Breite eines Unterbündels bezeichnet, dann gilt:

$$L \cdot \tan\theta \geq 0.76p - 0.63W.$$

5. Objektinformationserhaltevorrichtung mit:

einer Lichtquelle (601);
einem Faserbündel (602) mit einer Vielzahl von optischen Fasern; und
einem Prozessor, der eine akustische Welle, die erzeugt wird, wenn Licht von der Lichtquelle eine bestrahlte Oberfläche eines Objekts aus einer Ausgabeeinheit des Faserbündels bestrahlt, verwendet, und eine Information aus dem Objekt erhält, wobei
die Vielzahl der optischen Fasern so angebracht sind, dass eine Lichtmenge pro Einheitsfläche auf der bestrahlten Oberfläche, wenn sie mit Licht bestrahlt wird, nahezu einheitlich ist, wobei
Ausgabeebenen einer Vielzahl von Unterbündeln in der Ausgabeeinheit des Faserbündels angebracht sind, wobei jedes Unterbündel eine Vielzahl von optischen Fasern enthält,
die Vielzahl der Unterbündel ein erstes Unterbündel (603c), das in einem zentralen Teil der Ausgabeeinheit angebracht ist, und ein zweites Unterbündel (603a, 603b), das in einem peripheren Teil der Ausgabeeinheit angebracht ist, enthält,
**dadurch gekennzeichnet, dass**
eine Fläche der Ausgabeebene des ersten Unterbündels kleiner als eine Fläche der Ausgabeebene des zweiten Unterbündels ist.

6. Objektinformationserhaltevorrichtung nach Anspruch 5, wobei
die Anzahl der optischen Fasern pro Einheitsfläche die gleiche unter der Vielzahl der Unterbündel ist.

7. Objektinformationserhaltevorrichtung mit:

einer Lichtquelle (701);
einem Faserbündel (702) mit einer Vielzahl von optischen Fasern; und

einem Prozessor, der eine akustische Welle, die erzeugt wird, wenn Licht von der Lichtquelle eine bestrahlte Oberfläche eines Objekts von einer Ausgabeeinheit des Faserbündels bestrahlt, verwendet und eine Information aus dem Objekt erhält, wobei

die Vielzahl der optischen Fasern so angebracht sind, dass eine Lichtmenge pro Einheitsfläche der bestrahlten Oberfläche, wenn sie mit Licht bestrahlt wird, nahezu einheitlich ist, wobei

Ausgabekanten einer Vielzahl von Unterbündeln (703) in der Ausgabeeinheit des Faserbündels angebracht sind, wobei jedes Unterbündel eine Vielzahl von optischen Fasern enthält,

die Vielzahl der Unterbündel mehrere erste Unterbündel, die in einem zentralen Teil der Ausgabeeinheit angebracht sind, und mehrere zweite Unterbündel, die in einem peripheren Teil der Ausgabeeinheit angebracht sind, enthält,

**dadurch gekennzeichnet, dass**

die Ausgabekanten der ersten Unterbündel zerstreuter als die Ausgabekanten der zweiten Unterbündel in der Ausgabeeinheit angebracht sind.

## Revendications

1.  Appareil d'acquisition d'informations d'objet comprenant :

    une source de lumière (101) ;
    un faisceau de fibres (102) comprenant une pluralité de fibres optiques ; et
    un processeur qui utilise une onde acoustique qui est générée lorsque la lumière qui provient de la source de lumière est irradiée sur une surface irradiée d'un objet depuis une unité de délivrance du faisceau de fibres, et qui acquière les informations de l'objet, où
    la pluralité de fibres optiques est disposée de sorte qu'une quantité de lumière par unité de surface sur la surface irradiée lorsque la lumière est irradiée soit approximativement uniforme, où
    des bords de délivrance d'une pluralité de sous-faisceaux, chaque sous-faisceau comprenant une pluralité de fibres optiques, sont disposés dans l'unité de délivrance du faisceau de fibres,
    la pluralité de sous-faisceaux comprend un premier sous-faisceau (103b) disposé dans une partie centrale de l'unité de délivrance et un second sous-faisceau (103a) disposé dans une partie périphérique de l'unité de délivrance,
    **caractérisé en ce que**
    le nombre de fibres optiques par unité de surface du premier sous-faisceau est inférieur au nombre de fibres optiques par unité de surface du second sous-faisceau.

2.  Appareil d'acquisition d'informations d'objet selon la revendication 1, dans lequel
    les bords de délivrance de la pluralité de sous-faisceaux possèdent une taille identique et sont disposés à un même intervalle dans l'unité de délivrance.

3.  Appareil d'acquisition d'informations d'objet selon la revendication 2, dans lequel
    les bords de délivrance de la pluralité de sous-faisceaux sont disposés en forme de polygone,
    le second sous-faisceau comprend un sous-faisceau latéral qui est disposé sur un côté du polygone et un sous-faisceau de sommet qui est disposé sur un sommet du polygone, et
    les nombres de fibres optiques par unité de surface de la pluralité de sous-faisceaux sont dans un ordre exprimé comme suit :

    ```
    sous-faisceau de sommet > sous-faisceau latéral >
    premier sous-faisceau.
    ```

4.  Appareil d'acquisition d'informations d'objet selon la revendication 3, dans lequel
    lorsque L indique une distance entre le bord de délivrance du sous-faisceau et la surface irradiée, $\theta$ indique un angle sur lequel l'intensité de la lumière irradiée par la fibre optique passe d'une intensité maximale à $1/e^2$, p indique un intervalle minimum entre deux sous-faisceaux adjacents, et W indique une largeur d'un sous-faisceau, alors

$$L \cdot \tan\theta \geq 0.76p - 0.63W$$

est satisfait.

5. Appareil d'acquisition d'informations d'objet comprenant :

une source de lumière (601) ;
un faisceau de fibres (602) comprenant une pluralité de fibres optiques ; et
un processeur qui utilise une onde acoustique qui est générée lorsque la lumière qui provient de la source de lumière est irradiée sur une surface irradiée d'un objet depuis une unité de délivrance du faisceau de fibres, et qui acquière les informations de l'objet, où
la pluralité de fibres optiques est disposée de sorte qu'une quantité de lumière par unité de surface sur la surface irradiée lorsque la lumière est irradiée soit approximativement uniforme, où
des plans de délivrance d'une pluralité de sous-faisceaux, chaque sous-faisceau comprenant une pluralité de fibres optiques, sont disposés dans l'unité de délivrance du faisceau de fibres,
la pluralité de sous-faisceaux comprend un premier sous-faisceau (603c) disposé dans une partie centrale de l'unité de délivrance et un second sous-faisceau (603a, 603b) disposé dans une partie périphérique de l'unité de délivrance,
**caractérisé en ce que**
une surface du plan de délivrance du premier sous-faisceau est inférieure à une surface du plan de délivrance du second sous-faisceau.

6. Appareil d'acquisition d'informations d'objet selon la revendication 5, dans lequel
le nombre de fibres optiques par unité de surface est le même parmi la pluralité de sous-faisceaux.

7. Appareil d'acquisition d'informations d'objet comprenant :

une source de lumière (701) ;
un faisceau de fibres (702) comprenant une pluralité de fibres optiques ; et
un processeur qui utilise une onde acoustique qui est générée lorsque la lumière qui provient de la source de lumière est irradiée sur une surface irradiée d'un objet depuis une unité de délivrance du faisceau de fibres, et qui acquière les informations de l'objet, où
la pluralité de fibres optiques est disposée de sorte qu'une quantité de lumière par unité de surface sur la surface irradiée lorsque la lumière est irradiée soit approximativement uniforme, où
des bords de délivrance d'une pluralité de sous-faisceaux (703), chaque sous-faisceau comprenant une pluralité de fibres optiques, sont disposés dans l'unité de délivrance du faisceau de fibres,
la pluralité de sous-faisceaux comprend plusieurs premiers sous-faisceaux disposés dans une partie centrale de l'unité de délivrance et plusieurs seconds sous-faisceaux disposés dans une partie périphérique de l'unité de délivrance,
**caractérisé en ce que**
les bords de délivrance des premiers sous-faisceaux sont disposés de manière plus éparse que les bords de délivrance des seconds sous-faisceaux dans l'unité de délivrance.

FIG. 1

FIG. 2A

FIG. 2B

FIG. 2C

FIG. 2D

FIG. 2E

FIG. 3A

FIG. 3B

FIG. 3C

FIG. 3D

FIG. 3E

FIG. 4A

FIG. 4B

FIG. 4C

FIG. 4D

FIG. 5A

FIG. 5B

FIG. 5C

FIG. 5D

FIG. 5E

FIG. 6A

FIG. 6B

FIG. 6C

FIG. 6D

FIG. 7A

FIG. 7B

FIG. 7C

FIG. 7D

FIG. 8A

FIG. 8B

FIG. 8C

FIG. 8D

FIG. 9

FIG. 10A

FIG. 10B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002214707 A
- WO 2011012274 A1

- WO 2011135820 A1

**Non-patent literature cited in the description**

- **M. XU ; L. WANG.** Photoacoustic imaging in biomedicine. *Review of scientific instruments,* 2006, vol. 77, 041101